# EUROPEAN PATENT APPLICATION

(11) **EP 3 550 390 A1**
(43) Date of publication of application: **09.10.2019**
(21) Application number: 17876995.6
(22) Date of filing: 01.12.2017
(51) Int. Cl.: G05B 19/418, A61F 13/15, G05B 23/02

(54) **MANUFACTURING LINE CONTROL SYSTEM, MANUFACTURING LINE CONTROL DEVICE, AND MANUFACTURING LINE CONTROL METHOD**

(30) Priority: 02.12.2016 JP 2016235063; 27.11.2017 JP 2017226718
(71) Applicant: Kao Corporation, Chuo-Ku Tokyo 103-8210 (JP)
(72) Inventor: UENO, Kota, Haga-gun Tochigi 321-3497 (JP); KOKUBO, Makoto, Haga-gun Tochigi 321-3497 (JP); TAIRA, Naohiro, Haga-gun Tochigi 321-3497 (JP); MORITA, Akio, Haga-gun Tochigi 321-3497 (JP)
(74) Representative: Vossius & Partner Patentanwälte Rechtsanwälte mbB
(86) International application number: PCT/JP2017/043265
(87) International publication number: WO 2018/101457

(57) **Abstract**

A control system 10 of a production line, containing a processing unit 20 from which a workpiece 101 processed is obtained,
wherein the control system 10 has:
the processing unit 20; and
a control unit 11 for controlling the processing unit 20,
wherein the processing unit 20 has a plurality of unitary units 30,
wherein each of the plurality of unitary units 30 has a unitary unit identification code 1 to 8,
wherein the control system 10 of a production line has an inspection section 40 for inspecting the workpiece 101 processed,
wherein the inspection section 40 can communicate inside the control system 10 of a production line, and
wherein the inspection section 40 has an evaluation section 50 in which state of the processed workpiece 101 is evaluated to obtain evaluation results; a data processing section 60 in which the evaluation results are associated with the unitary unit identification codes 1 to 8 of the processing unit 20 in which the evaluated workpiece 101 is processed; a storage section 71 in which the evaluation results are stored for each of the unitary unit identification codes 1 to 8; and a reporting section 81 by which the unitary unit identification codes 1 to 8 and the evaluation results are reported.

## Description

### FIELD OF THE INVENTION

The present invention relates to a control system of a production line, a control device of a production line, and a method of controlling a production line, in which an abnormal site is promptly detected.

### BACKGROUND OF THE INVENTION

When an abnormal product or a defective product occurs among products produced on a production line, prompt investigation of cause is often required. Examples include cases where a defective product is found during quality inspection in the production process and cases where complaints are received from consumers. Moreover, also when an equipment malfunction occurs in a production apparatus, prompt investigation of cause is similarly required.

Specifically, it is required to detect occurrence of poor processing in the production line in order to pinpoint the site of occurrence. Patent Literature 1 discloses a system in which a form image of an absorbent article is obtained and defects of the absorbent article are detected by examining the form image, whereby site of occurrence can be determined.

### CITATION LIST

### PATENT LITERATURES

Patent Literature 1: JP-A-2013-013523 ("JP-A" means unexamined published Japanese patent application)

### SUMMARY OF THE INVENTION

The present invention provides a control system of a production line, containing a processing unit from which a workpiece processed is obtained, wherein the control system has:
the processing unit; and
a control unit for controlling the processing unit,
wherein the processing unit has a plurality of unitary units,
wherein each of the plurality of unitary units has a unitary unit identification code,
wherein the control system of a production line has an inspection section for inspecting the workpiece processed,
wherein the inspection section can communicate inside the control system of a production line, and
wherein the inspection section has an evaluation section in which state of the processed workpiece is evaluated to obtain evaluation results; a data processing section in which the evaluation results are associated with the unitary unit identification codes of the processing unit in which the evaluated workpiece is processed; a storage section in which the evaluation results are stored for each of the unitary unit identification codes; and a reporting section by which the unitary unit identification codes and the evaluation results are reported.

The present invention provides a control device of a production line, containing a processing unit from which a workpiece processed is obtained, wherein the control device has:
the processing unit; and
a control unit for controlling the processing unit,
wherein the processing unit has a plurality of unitary units,
wherein each of the plurality of unitary units has a unitary unit identification code,
wherein the control unit has an inspection section for inspecting the workpiece obtained from the processing unit,
wherein the inspection section can communicate inside the control system of a production line, and
wherein the inspection section has an evaluation section in which state of the processed workpiece is evaluated, to obtain evaluation results; a data processing section in which the evaluation results are associated with the unitary unit identification codes of the processing unit in which the evaluated workpiece is processed; a storage section in which the evaluation results are stored for each of the unitary unit identification codes; and a reporting section in which the unitary unit identification codes and the evaluation results are reported.

The present invention provides a method of controlling a production line having a plurality of processing units for producing a workpiece, containing the steps of:
assigning a unitary unit identification code to each of a plurality of unitary units of the processing unit;
inspecting the produced workpiece;
evaluating state of the inspected workpiece to obtain evaluation results;
associating the evaluation results with the unitary unit identification codes of the processing unit in which the evaluated workpiece is processed;
storing the evaluation results for each of the unitary unit identification codes; and
reporting the unitary unit identification codes and the evaluation results.

Other and further objects, features and advantages of the invention will appear more fully from the following description, appropriately referring to the accompanying drawings.

### BRIEF DESCRIPTION OF DRAWINGS

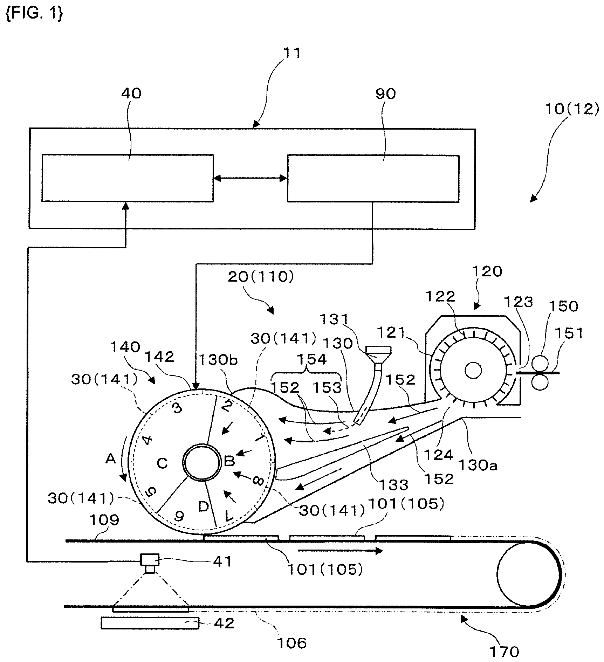

{FIG. 1}
   FIG. 1 is a schematic configuration diagram showing one preferable example of a control system of a production line according to the present invention.
{FIG. 2}
   FIG. 2 is a block diagram showing one preferable example of the inspection section shown in FIG. 1.
{FIG. 3}
   FIG. 3 is a diagram showing one example of images of absorbent bodies including absorbent bodies in which minute chipping occurs, which are aligned for each unitary unit identification code.
{FIG. 4}
   FIG. 4 is a graph showing one example of a relationship between the number of black pixels and the number of pieces evaluated in images of absorbent bodies by applying unitary unit identification codes as a parameter.
{FIG. 5}
   FIG. 5 is a diagram showing one example of images of absorbent bodies including absorbent bodies having a small basis weight, which are aligned for each unitary unit identification code.
{FIG. 6}
   FIG. 6 is a graph showing one example of a relationship between the average gradation value and the number of pieces evaluated in images of absorbent bodies by applying unitary unit identification codes as a parameter.
{FIG. 7}
   FIG. 7 is a schematic perspective view showing one example of precutting of an elastic body.
{FIG. 8}
   FIG. 8 is plan views showing one example of poor precutting of an elastic body, in which FIG. 8(A) is a plan view showing one example of a state in which a part of the elastic bodies is not precut, and FIG. 8(B) is a plan view showing one example of a state in which none of the elastic bodies is precut.
{FIG. 9}
   FIG. 9(A) is a plan view showing one example of a state in which an elastic body is normally precut, FIG. 9(B) is an enlarged view of the image-pickup area shown in FIG. 9(A), and FIG. 9(C) is an enlarged view of an inspection region shown in FIG. 9(B).
{FIG. 10}
   FIG. 10 is a front view showing one example of ultrasonic welding equipment.
{FIG. 11}
   FIG. 11 is a partial front view showing a part of a production line having a plurality of processing units.
{FIG. 12}
   FIG. 12 is a plan view showing one example of a picked-up image and a measurement site of an absorbent body.

### DESCRIPTION OF EMBODIMENTS

The present invention relates to a control system, a control device, and a method of controlling a production line that enable prompt detection of abnormal sites. The abnormal product or the defective product mentioned above occurs in a multiple-piece unit used in an absorbent article production apparatus (the term "multiple-piece unit" usually means a unit such as has a plurality of unitary units for performing the same processing) in several cases. The present invention relates to a technology in which, when any abnormality occurs in one of such multiple-piece units, the abnormal site is promptly pinpointed. Moreover, the present invention relates to a technology in which time required for adjusting and monitoring the production apparatus in order to eliminate problems is shortened, thereby reducing maintenance burden.

The present invention enables prompt detection of abnormal sites and improves production time-efficiency of the production apparatus by reducing time spent on examining units requiring no maintenance. Moreover, it enables small difference in evaluation results arising among specific unitary units to be ascertained from unitary unit identification code specific data. Accordingly, an operator of the production line can easily perform adjustment for inhibiting product variance, whereby quality improvement of the workpiece can be realized.

One preferable embodiment of the control system of a production line according to the present invention will be described below by taking a method of producing an absorbent article as one example with reference to drawings.

As shown in FIG. 1, the control system 10 of a production line according to the present invention is a control system having a processing unit 20 of a production line from which workpieces 101 are obtained. Hereinafter, the "control system 10 of a production line" is sometimes referred to simply as the "control system 10." The production line ordinarily has a plurality of processing units 20. Hereinafter, the production line will be described with focus on a single processing unit 20. The aforesaid term "processing" means processing utilizing physical action, chemical action, physical-chemical action or the like, including deformation processing, addition processing, removal processing, special processing or the like, or processing by combination of the aforesaid types of processing. Examples of the deformation processing include fiber-stacking, compression and rolling, examples of the addition processing include welding, bonding and coating, and examples of the removal processing include shearing and cutting. Moreover, examples of the special processing include ultrasonic irradiation, laser irradiation and plasma irradiation. Moreover, changing of article positions is categorized into the processing as displacement processing. However, the examples of processing cited above are not comprehensive, and processing is not limited to the examples of processing listed above.

The processing unit 20 has a plurality of unitary units 30. FIG. 1 shows an absorbent body production apparatus 110 as the processing unit 20. The term "unitary unit 30" means one among a plurality of fiber-stacking recesses 141 in a fiber stacker 140 of the production apparatus 110, and unitary units identical processing elements.

Each of the unitary units 30 assigns a unitary unit identification code. The term "unitary unit identification code" means a code assigned to the unitary unit 30 forming the processing unit 20 and used to identify the unitary unit 30. The codes can be numbers, letters, combinations of numbers and letters or of any other type insofar capable of identifying the unitary units. The codes are ordinarily a series of natural numbers starting from 1, or a series of positive integers starting from 0, or expressed as a series of letters in alphabetic order, such as "A", "B", "C", .... The unitary unit identification codes in the illustrated example consist of the natural numbers from 1 to 8 in the order of, for example, "1", "2", ..., "8". The aforesaid unitary unit identification codes are preferably assigned to the unitary unit 30 in advance.

Moreover, the control system 10 has a control unit 11 including an inspection section 40 for inspecting the workpieces 101 processed by the processing unit 20. The control unit 11 is preferably equipped with a control section 90 described later.

The inspection section 40 has an image pickup section 41 for picking up an image of each workpiece 101 processed by the processing unit 20, and a lighting section 42 for illuminating the workpiece 101. When the image is picked up utilizing transmitted artificial light, as shown in the figure, the lighting section 42 is arranged to face the image pickup section 41 in imaging direction from opposite side of the workpiece 101, which is the object to be imaged. The image pickup section 41 is arranged at a position where L sheets are conveyed after processing by the processing unit 20. Moreover, although not shown, when the image is picked up utilizing reflected artificial light, the lighting section 42 is arranged on same side of the image pickup object (workpiece 101) as the image pickup section 41 at a position so as not to block field of view from the image pickup section 41 toward the workpiece 101.

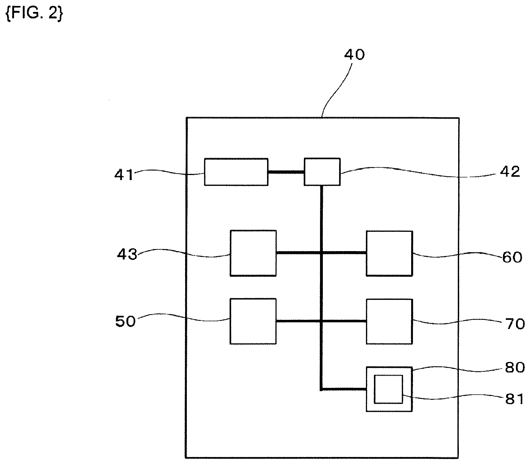

Further, as shown in FIG. 2, the inspection section 40 has an image processing section 43, an evaluation section 50, a data processing section 60, a storage section 70 and a reporting section 80. Moreover, the inspection section 40 is adapted to enable intercommunication among individual sections, including the processing unit 20, in the control system 10 (see FIG. 1). The inspection section 40 is configured to intercommunicate with at least the control section 90.

Operation of the respective sections of the inspection section 40 will now be described.

The image processing section 43 processes images picked up by the image pickup section 41 with respect to preset content to thereby obtain processed data. The term "processed data" means data obtained as a result of image processing, including, for example, images obtained by performing various types of filtering (for example, binarization processing, averaging processing, expansion processing, contraction processing and the like) of the picked-up images. Alternatively, the processed data is numerical data such as coordinate data, the number of pieces, and degree of coincidence, which are obtained by various types of characteristic extraction processing (area measurement, the number of blobs, edge detection processing, pattern matching processing and the like). Alternatively, the processed data is a picked-up image, an image subjected to various types of filtering, or a combination of numerical data obtained by various types of characteristic extraction processing.

The evaluation section 50 evaluates state of the workpiece produced by the processing unit by comparing processed data obtained by the image processing section 43 and pre-registered normal product processed data. That is, the evaluation section 50 receives and uses processed data obtained by the image processing section 43 to evaluate with respect to predefined thresholds whether or not the imaged workpiece 101 is normal, and outputs quality evaluation data to the control section 90 (see FIG. 1).

The data processing section 60 associates the evaluation results obtained by the evaluation section 50 with the unitary unit identification code of the unitary unit 30 in which the evaluated workpiece 101 was processed. The term "evaluation results" means data to be used for evaluating in the evaluation section 50. Examples of this data can include picked-up image, binarized image, the number of counted pixels, quality evaluation data, serial number, and production date and production time of the workpiece 101 when the workpiece 101 was processed.

The storage section 70 stores the evaluation results for each unitary unit identification code. The volume of data to be stored is not particularly limited. Capacity of a storage medium (not shown) to be used in the storage section 70 should be decided based on an estimate of data capacity taking required data retention period into consideration. Moreover, the volume of data to be retained can also be changed according to kind of data by setting a larger volume of retained numerical data (production date and time, the number of counted pixels), or reducing the number of large data volume images retained.

The reporting section 80 reports the unitary unit identification codes and the evaluation results. The reporting section 80 has a display section 81 for displaying the unitary unit identification codes associated with the evaluation results, and the evaluation results. The reporting section 80 uses the display section 81 to display whether the workpiece is normal or abnormal on a screen thereof at appropriately set times, such as every time the workpiece 101 is processed, every hour, and every day. Thus, the reporting section 80 informs the operator of operating state of the processing unit. Alternatively, the display section 81 can also display trends of the evaluation results over appropriately set periods.

Further, the reporting section 80 preferably has a sound output section (not shown) for reporting abnormalities by a warning tone or sound together with a screen display. For example, the reporting section 80 may be configured to report the unitary unit identification codes associated with the evaluation results, and the evaluation results by sound. In addition, the reporting section 80 can also have a printing section (not shown). The printing section can also output the evaluation results for each unitary unit identification code by filling out a form at appropriately set times, such as every hour, every day, and every month.

The control unit 11 includes the control section 90. The control section 90 can communicate inside the control system 10. Accordingly, the control section 90 and the inspection section 40 are connected to communicate with each other. The control section 90 is responsive to evaluation results associated with the unitary unit identification codes for, as required, issuing instructions to change production conditions of the processing unit 20 or to stop the production line.

The production line preferably additionally includes an ejection unit (not shown) for ejecting workpieces 101 evaluated to be defective by the evaluation section 50 to outside of the production line. The control system 10 or the control unit 11 controls the ejection unit on the basis of the quality evaluation data received from the evaluation section 50. That is, when the quality evaluation data indicate a defective product, the control system 10 or the control unit 11 activates an ejection mechanism (not shown) of the ejection unit when the defective product passes through the ejection unit, whereby the defective product can be ejected from the production line.

The above-described control system 10 can promptly and accurately detect abnormal sites. Therefore, among the plurality of processing units 20, time spent on examining those processing units 20 that require no maintenance can be reduced, whereby production time-efficiency of the production apparatus can be improved. Moreover, small difference in evaluation results arising among individual unitary units associated with identification codes can be ascertained. Accordingly, the operator of the production line can easily adjust the processing conditions and the like for inhibiting product variance, whereby the quality of the workpiece can be improved.

A control device 12 in the production line in which the above-described control system 10 is utilized incorporates the aforesaid plurality of processing units 20 and the control unit 11 for controlling the processing units 20.

The processing unit 20 has the plurality of unitary units 30, and each of the plurality of unitary units has the unitary unit identification code. On the other hand, the control unit 11 has the inspection section 40 for inspecting the workpiece 101. The inspection section 40 is formed to enable communication between sections inside the control device 12, such as between the inspection section 40 and the control section 90 of the control unit 11, or between the control unit 11 and the processing unit 20.

As mentioned above, the inspection section 40 has the evaluation section 50, the data processing section 60, the storage section 70, and the reporting section 80. The inspection section 40 further preferably incorporates the image processing section 43 having the image pickup section 41 and the lighting section 42 for acquiring information (shape, thickness or the like) of the workpiece 101.

Moreover, the control unit 11 preferably incorporates the control section 90 responsive to evaluation results of the evaluation section 50 corresponding to the unitary unit identification codes for, as required, issuing instructions to change production conditions of the processing unit 20 or to stop the production line.

The method of controlling a production line upon producing the workpiece 101 using the above-described control system 10 of a production line (control device 12 of a production line) will be described.

Each of the plurality of unitary units 30 of the processing unit 20 is assigned the unitary unit identification code (step 1). Owing to this step 1, the unitary units 30 can be easily identified even if a plurality of similar unitary units 30 are provided.

The individual workpieces produced in the processing unit 20 are then inspected (step 2). In the control system 10, this step 2 is performed in the inspection section 40. In the inspection section 40, processing as mentioned above is performed by the image processing section 43 on the basis of images picked up in the image pickup section 41.

Whether or not the state of each inspected workpiece is normal is evaluated to obtain evaluation results (step 3). In the step 3, in which evaluation results are obtained, the picked-up image obtained by picking up the image of the workpiece 101 produced by the processing unit 20, by the image pickup section 41, is subjected to image processing by the image processing section 43. The processed data obtained by this image processing is compared with the pre-registered normal product processed data to evaluate the state of the workpiece 101 produced by the processing unit 20.

The evaluation results are assigned in correspondence to the unitary unit identification codes of the processing unit 20 in which the workpiece 101 evaluated by the evaluation section 50 is processed (step 4). In the control system 10, this step 4 is performed in the data processing section 60. Thus, each workpiece evaluated to be normal or abnormal is associated with the unitary unit 30 having the unitary unit identification code in which the workpiece was processed.

Then, the evaluation results are stored for each unitary unit identification code (step 5). In the control system 10, this step 5 is performed in the storage section 70.

The unitary unit identification codes and the evaluation results are reported (step 6). In the control system 10, this step 6 is performed in the reporting section 80. Specifically, the unitary unit identification codes associated with the evaluation results, and the evaluation results are displayed by the reporting section 80. As mentioned above, various methods can be applied for performing this display, such as displaying the information on the display section 81, reporting by warning tone or sound, and outputting the information as a printed form.

Particulars regarding the processing unit 20, the unitary unit 30, the unitary unit identification code, the inspection section 40, the evaluation section 50, the data processing section 60, the storage section 70 and the reporting section 80 are as described above.

Further, the unitary unit identification codes and the evaluation results can be transmitted to the control section 90, and the control section 90 can be adapted to issue instructions to change the production conditions of the processing unit 20 or to stop the production line on the basis of the evaluation results (in particular, evaluation results indicating abnormality) corresponding to the unitary unit identification codes. Moreover, the defective product can be ejected from the production line. Thus, the plurality of processing units 20 can be controlled.

Hereinafter, the control system, the control device and the method of controlling a production line will be described on the basis of specific embodiments.

First, an absorbent body production apparatus 110 shown in FIG. 1 will be described in detail below. An absorbent body production apparatus 110 supplies an absorbent material 154 containing a fiber material, together with airflow, through an inside of a duct 130, and deposits the absorbent material 154 into a recess (hereinafter, also referred to as a fiber-staking recess) 141 arranged on a peripheral surface of a rotating drum 142 of a fiber stacker 140.

The production apparatus 110 has a pulp feeder 150 for paying out a pulp sheet 151 drawn from a pulp stock (not shown), and a defibrator 120 for defibrating a pulp sheet 151 to obtain the pulp fiber 152. Further, the production apparatus 110 has the duct 130 serving as a route for carrying and conveying on the airflow the pulp fiber 152 delivered from the defibrator 120.

The defibrator 120 has a casing 121, a rotating blade 122 disposed in the casing 121 to scratch an end portion of the pulp sheet 151 for making it fibrous. The casing 121 has an opening portion 123 provided in the casing 121 to introduce the pulp sheet 151 therefrom, and an opening portion 124 for discharging the pulp fiber 152 therefrom.

One end portion 130a of the duct 130 is connected to the opening portion 124 of the defibrator 120 and the other end portion 130b thereof partially covers the peripheral surface of the rotating drum 142.

In the rotating drum 142, for example, a plurality of fiber-stacking recesses 141 are formed on a circumferential surface at a predetermined interval. Toward the circumferential surface of this rotating drum 142, the absorbent material 154 (the pulp fiber 152, the absorbent polymer 153) (shown by an arrow, for convenience) conveyed inside the duct 130 is supplied and deposited into the fiber-stacking recess 141.

The absorbent body 105 as a workpiece 101 stacked in the fiber-stacking recess 141 is used for the absorbent body of the absorbent article such as a disposable diaper for babies, a sanitary napkin and an incontinence pad. Consequently, a shape of the above-described fiber-stacking recess 141 is determined according to the shape of the absorbent body 105. More specifically, the shape of the fiber-stacking recess 141 is determined such that a convex portion and a concave portion may be formed in sites necessary for the absorbent body 105. In addition, the shape of the fiber-stacking recess 141 is not limited thereto, and depth may be fixed, or the recesses 141 may be continuously formed along the peripheral surface of the rotating drum 142.

To the rotating drum 142, a suction fan (not shown) is connected, and the space B in the rotating drum 142 is maintained in a negative pressure by working of the suction fan. This negative pressure in the space B generates the air flow in the duct 130 and the absorbent material 154 from the defibrator 120 is set in the flying state. At least a bottom portion of the individual fiber-stacking recesses 141 is configured by a mesh plate or the like, and has many pores. While the individual fiber-stacking recesses 141 pass through the space B maintained in the negative pressure, the pores of the mesh plate function as suction holes. The space B is located on a side reverse to a part covered with at least the duct 130 in the rotating drum 142. The space B generates strong suction force in the fiber-stacking recess 141 passing through the part covered with the duct 130 to stack the absorbent material 154 in the fiber-stacking recess 141, and generates the air flow conveying the absorbent material 154 in the duct 130. In order to convey a stacked material or the absorbent body while it is stably retained in the fiber-stacking recess 141, a space C may be maintained in the negative pressure, and in this case, the space C may be maintained in a negative pressure level lower than the level in the space B.

The airflow for conveying the absorbent material 154 flowing inside the above-described duct 130 is guided toward the peripheral surface of the rotating drum 142 by suction from the fiber-stacking recess 141 located above the space B.

Further, in a position in which the absorbent body 105 is peeled off from the fiber-stacking recess 141, pressure in a space D in the rotating drum 142 is increased than the pressure in the rotating drum 142, whereby releasability of the absorbent body 105 may be enhanced.

Further, the production apparatus 110 has a conveying device 170 as a transfer conveying mechanism for releasing the absorbent body 105 from the fiber-stacking recess 141 and transferring the absorbent body 105 onto a backing 109. Accordingly, a continuous body 106 having the absorbent bodies 105 arranged at an equal interval on the backing 109 is formed.

As the absorbent material 154, various kinds of materials to be used for the absorbent body of an absorbent article such as a sanitary napkin, a disposable diaper, or the like can be used without limitation, and the material 154 contains at least the fiber material. As the fiber material, for example, the pulp fiber obtained by defibrating the pulp sheet, and also a short fiber of a cellulose fiber such as a rayon fiber and a cotton fiber, a short fiber of a synthetic fiber of polyethylene, or the like can be used. These fiber materials can be used alone in one kind or in combination of two or more kinds. Moreover, as the absorbent material 154, the absorbent polymer can be further used.

As described above, an absorbent body 105 is processed by stacking fibers by using the fiber stacker 140. Then, the absorbent body 105 released from the fiber-stacking recess 141 on the rotating drum 142 in the fiber stacker 140 is conveyed by being placed on the backing 109. In the conveying process therefor, a shape of a fiber-stacked absorbent body 105 is inspected by the inspection section 40 of the control unit 11. In the inspection, the image pickup section 41 and the lighting section 42 of the inspection section 40 are used. The image pickup section 41 is arranged above an image pickup position of the absorbent body 105, and the lighting section 42 is arranged in a position facing the image pickup section 41 across the absorbent body 105. The absorbent body 105 is irradiated with light from a rear surface thereof by the lighting section 42 to pick up a transmitted image of the absorbent body using the image pickup section 41.

As the image pickup section 41, such a camera that can be applied to the production apparatus may be used, and for example, an image processing camera XG-HL02M (product name: manufactured by Keyence Corporation) can be used.

In the case of the absorbent body 105, the image processing section 43 binarizes the picked up image obtained by the image pickup section 41 using a preset binarized threshold T_{B}. A pixel less than the binarized threshold T_{B} is converted into black, and a pixel more than the binarized threshold T_{B} is converted into white, and the number of pixels converted into black is counted.

The evaluation section 50 judges whether or not the number of black pixels counted is within the range between a lower limit threshold T_{PL} and an upper limit threshold T_{PH} of the preset number of pixels to evaluate whether or not the picked up workpiece 101 is normal. The quality evaluation data is output to the data processing section 60.

In the data processing section 60, the evaluation results obtained by the evaluation section 50 are associated with the unitary unit identification codes 1 to 8 of the unitary unit 30 (fiber-stacking recess 141) in which the evaluated workpiece 101 (absorbent body 105) is processed.

### <Detection of minute chipping using moving average>

As a method for detecting any abnormality of the workpiece 101 (absorbent body 105), the following method will be disclosed in addition thereto.

In the storage section 70, the evaluation results of each absorbent body 105 provided with the unitary unit identification code are stored for each unitary unit identification code.

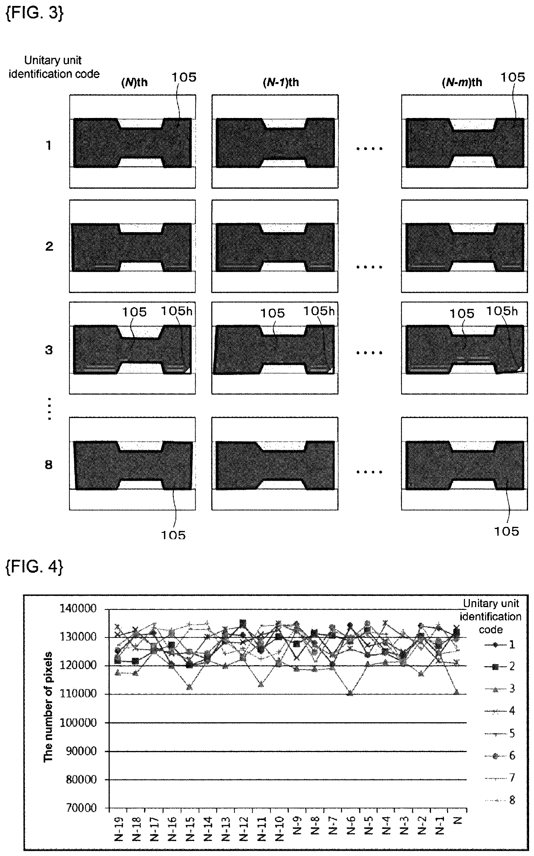

For example, as shown in FIG. 3, the evaluation results from (N)th to (N-m)th are stored for each number in the unitary unit identification codes 1 to 8. N and m satisfy an inequality: N > m, and both are a natural number.

The results are as shown in FIG. 4 on a graph of the number of black pixels and the number of pieces evaluated by applying the unitary unit identification codes as a parameter. As is apparent from FIG. 4, it was found that a great number of the absorbent bodies 105 having the number of black pixels smaller than the average number of black pixels exist in the absorbent bodies 105 processed by the unitary unit 30 (fiber-stacking recess 141) having the unitary unit identification code 3. However, the number of black pixels for the unitary unit identification code in the normal product is also fluctuated. For example, when (N-1)th data in FIG. 4 is viewed, the number of pixels of the unitary unit identification code 4 in the product having no abnormality is less than the number of black pixels of the code 3 in the product having any abnormality, and it was found to be difficult to merely evaluate the quality only by the thresholds (T_{PL} and T_{PH}) of the number of black pixels previously used.

As a method for evaluating the quality in such a case, as shown in Table 1, an average value of the number of black pixels of the absorbent bodies 105, for example, 20 pieces of the absorbent bodies 105, which are processed by the fiber-stacking recesses 141 having the unitary unit identification codes for each, can be used for each of the unitary unit identification codes 1 to 8. This number of pieces is not limited to 20, and as the number of pieces increases, accuracy is statistically higher, but a time excessively takes for obtaining the number of data necessary for evaluation from an initial stage of operation, and therefore such a case is not preferable. For example, the number of pieces is 2 or more, preferably 5 or more, and more preferably 10 or more, and 100 or less, and preferably 50 or less, and more preferably 30 or less.

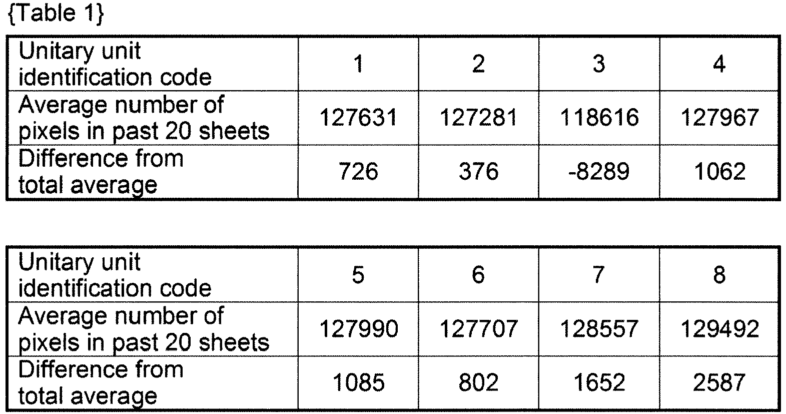

As is apparent from Table 1 described above, it was found that an average value of the number of black pixels of the absorbent bodies 105 processed by the fiber-stacking recess 141 having the unitary unit identification code 3 is smaller than a total average value of the number of black pixels of the absorbent bodies 105 processed by the fiber-stacking recesses 141 having the unitary unit identification codes 1 to 8. This finding indicates that minute chipping 105h exists in the absorbent bodies 105 processed by the fiber-stacking recess 141 having the unitary unit identification code 3.

The data processing section 60 calculates the total average value ((N-m) × n pixels) of the number of black pixels and the average value of the number of black pixels for each unitary unit identification code (n is a natural number). Then, in the case of Table 1, under an assumption: n = 8, the data processing section 60 determines a differential between the total average value of the number of black pixels and the average value of the number of black pixels in each unitary unit identification code ([total average value] - [average value in each unitary unit]).

The evaluation section 50 judges whether or not the differential value for each unitary unit identification code is within the range of a preset lower limit threshold TS_{PL} and a preset upper limit threshold TS_{PH}. The evaluation results are stored for each unitary unit identification code. This storage is performed in the storage section 70. When the differential value for each unitary unit identification code is out of the range, any abnormality is judged to occur to transmit a minute chipping occurrence flag and the unitary unit identification code of the unitary unit 30 (fiber-stacking recess 141) in which the minute chipping occurs to the reporting section 80 and the control section 90 as detected information. The reporting section 80 displays whether the workpiece is normal or abnormal on the screen of the display section 81. Moreover, in the case of any abnormality, a message based on the detected information is displayed, whereby the operator can easily know an abnormality occurrence site and a content of any abnormality.

The control section 90 can also allow the production line to stop when any abnormality occurs by the same unitary unit continuously in the number of times equal to or more than the preset number of times on the basis of the detected information transmitted from the inspection section 40. After the production line is stopped, the operator inspects the unitary unit 30 in which any abnormality occurs. For example, when the minute chipping occurs as described above, after the production line is stopped, the operator inspects the fiber-stacking recess 141 having the unitary unit identification code in which the minute chipping occurs, and eliminates a cause of occurrence. Alternatively, the control section 90 can allow the production conditions of the processing unit 20 requiring a change in the production conditions to change. Thus, the above-described control system 10 (control device 12) promptly and accurately detects the abnormal site, and therefore any abnormality can be eliminated.

Next, a case where a basis weight of the absorbent body 105 has any abnormality will be described below.

For example, when the basis weight of the absorbent body 105 is small, according to the image processing section 43, a picked-up image of such an absorbent body 105 is picked up as an image having a high gradation. Such an image of the absorbent body is picked up at brightness higher than other images of the absorbent bodies. The image picked up at brightness higher than other images in this manner is produced owing to a larger quantity of transmitted light in the part than in a surrounding, which means that the basis weight of the absorbent body 105 is smaller.

Thus, when the basis weight of the absorbent body 105 has any abnormality, the picked-up image picked up by the image pickup section 41 is picked up as an image having a gradation different from other picked-up images of normal basis weight absorbent bodies.

This finding is utilized, whereby a calibration curve representing a relationship between the basis weight and a gradation value of the absorbent body 105 is preliminary prepared, and an allowable range of the gradation value can be determined from an allowable range of the basis weight of the absorbent body 105.

Specifically, the image processing section 43 presets such an inspection region R as generally covering all the absorbent bodies 105, and picks up the images of the absorbent bodies 105 while changing the basis weights of the absorbent bodies 105 to obtain the picked-up images. The image processing section 43 calculates an average gradation value within the region R for each picked-up image obtained by picking up the images of the absorbent bodies 105 to preliminarily prepare the calibration curve representing the relationship between the basis weight and the gradation value of the absorbent body 105. The allowable range of the above-described gradation value (average gradation value) is determined from the allowable range of the basis weight of the absorbent body 105 on the basis of the prepared calibration curve.

The evaluation section 50 evaluates such a case normal when the value is within the range between a preset lower limit threshold T_{GL} of the average gradation value and a preset upper limit threshold T_{GH} thereof, and such a case abnormal in the basis weight of the absorbent body when the value is out of the range. Then, the data processing section 60 allows the evaluation results to correspond to a basis weight abnormality flag, and the unitary unit identification code in which any basis weight abnormality occurs, and to store the correspondence in the storage section 70. At the same time, the correspondence is transmitted to the reporting section 80 and the control section 90 as the detected information.

### <Detection method for minute basis weight difference>

In addition thereto, the following is disclosed as a means for detecting a minute basis difference in the workpiece 101 (absorbent body 105).

In the storage section 70, as described above, the evaluation results of each absorbent body 105 provided with the unitary unit identification code are stored for each unitary unit identification code.

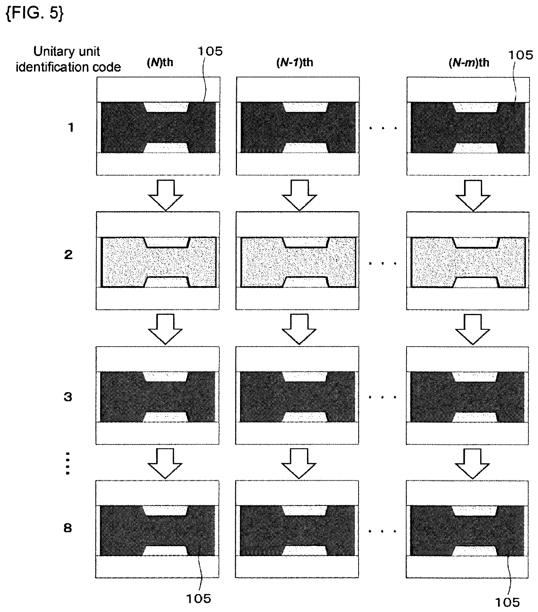

For example, as shown in FIG. 5, the evaluation results from (N)th to (N-m)th are stored for each number in the unitary unit identification codes 1 to 8. Here, N and m satisfy an inequality: N > m, and both are a natural number.

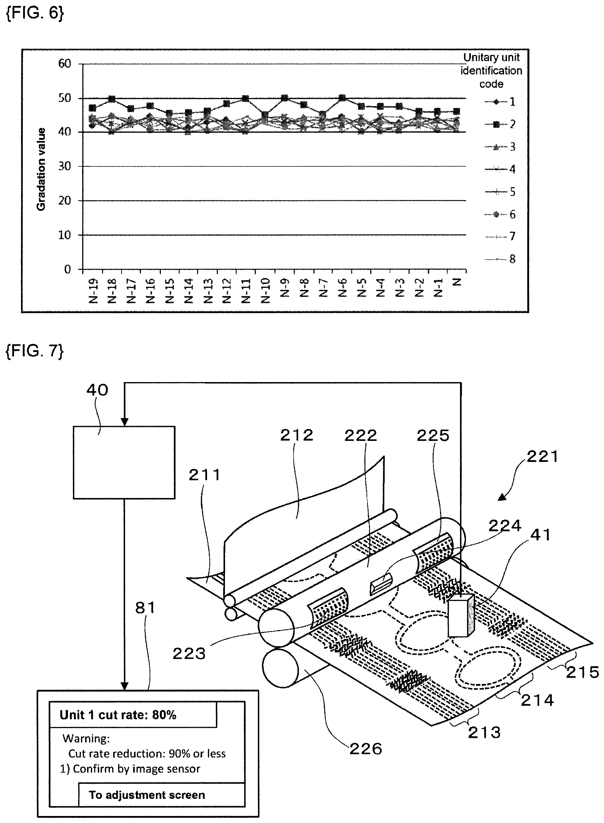

The results are as shown in FIG. 6 on a graph of the gradation values and the number of pieces judged by applying the unitary unit identification codes as the parameter. FIG. 6 shows that the gradation value of the image of the absorbent body 105 processed by the unitary unit 30 (fiber-stacking recess 141) having the unitary unit identification code 2 is higher than the average gradation value. Accordingly, it was found that the absorbent body 105 having the unitary unit identification code 2 has a smaller basis weight than the absorbent bodies 105 having any unitary unit identification codes other than the unitary unit identification code 2.

For example, Table 2 shows an average value of the gradation values of 20 pieces of the absorbent bodies 105, for example, which are processed by the fiber-stacking recess 141 having each unitary unit identification code for each of the unitary unit identification codes 1 to 8. This number of pieces is not limited to 20, and as the number of pieces increases, the accuracy is statistically higher, but if the number is excessively large, the time takes for obtaining the number of data necessary for judgment from the initial stage of operation. Therefore, it is necessary to appropriately examine the above-described number. For example, the number of pieces is 2 or more, preferably 5 or more, and more preferably 10 or more, and 100 or less, preferably 50 or less, and more preferably 30 or less.

**{Table 2}**

| Unitary unit identification code | 1 | 2 | 3 | 4 | 5 | 6 | 7 | 8 |
|---|---|---|---|---|---|---|---|---|
| Average gradation value in past 20 sheets | 43 | 47 | 42 | 43 | 42 | 42 | 42 | 43 |
| Difference from total average | 0 | -4 | 1 | 0 | 1 | 1 | 1 | 0 |

As is apparent from Table 2, it was found that the average gradation value of the absorbent bodies 105 processed by the fiber-stacking recess 141 having the unitary unit identification code 2 is higher than the total average gradation value of the absorbent bodies 105 processed by the fiber-stacking recesses 141 having the unitary unit identification codes 1 to 8. The finding indicates that the basis weight is reduced in the absorbent body 105 processed by the fiber-stacking recess 141 having the unitary unit identification code 2. In addition, the total average gradation value is an average gradation value of (N-m) × 8 pieces.

This finding is utilized to calculate a differential between the total average gradation value of the absorbent bodies 105 and the average gradation value in the unitary unit identification codes ([total average gradation value] - [average gradation value in each unitary unit]).

The evaluation section 50 judges whether or not the calculated differential value is out of a preset lower limit threshold TS_{GL} of a differential and a preset upper limit threshold TS_{GH} thereof. When the above-described differential value is out of the range of the thresholds, any abnormality is judged to occur. Then, the data processing section 60 allows the evaluation results to correspond to a minute basis weight abnormality flag and the unitary unit identification code of the unitary unit 30 in which any minute basis weight abnormality occurs, and to store the correspondence in the storage section 70. At the same time, the correspondence is transmitted to the reporting section 80 and the control section 90 as the detected information. The reporting section 80 displays whether the workpiece is normal or abnormal on the screen of the display section 81. Then, as described above, the message based on the detected information in the case of any abnormality is displayed thereon, whereby the operator can easily know the abnormality occurrence site and the content of any abnormality.

The control section 90 can allow a production condition of the processing unit 20 requiring the change in the production condition to change on the basis of the detected information transmitted from the inspection section 40. For example, when any basis weight abnormality occurs as described above, the control section 90 adjusts an amount of air during fiber stacking or a feed amount per unit time in the pulp feeder 150 as the production conditions, for example, to eliminate causes of occurrence of any basis weight abnormality. Alternatively, the control section 90 can also allow the production line to stop. For example, when any abnormality occurs in the unitary unit 30 having the same unitary unit identification code continuously in the number of times equal to or more than the preset number, the control section 90 allows the production line to stop. After the production line is stopped, the operator inspects the unitary unit 30 in which any abnormality occurs on the basis of the reported abnormality content, and repairs the abnormal site or exchange components in the abnormal site. Thus, the control system 10 (control device 12) can promptly and accurately detect the abnormal site of the unitary unit 30 and restore the site.

Next, ejection of the defective product will be specifically described.

For example, as shown in FIG. 3 mentioned above, consider a case where the minute chipping 105h occurs in the absorbent body 105 in the unitary unit identification code 3 by the fault of the production apparatus 110. Alternatively, as shown in FIG. 5 mentioned above, consider a case where a defect of a low basis weight of the absorbent body 105 occurs in the unitary unit identification code 2 by the fault of the production apparatus 110.

The inspection section 40 sequentially stores inspection data while inspecting the workpiece 101 (image pickup and image processing). If the inspection results are defective, a defective product signal is output from the control section 90 to an ejection unit (not shown). That is, the control section 90 allows the ejection unit to operate on the basis of the evaluation results of the evaluation section 50 to eject the defective product from the production line.

According to the control system 10 (control device 12) of the present invention, each of the plurality of unitary units 30 of the processing unit 20 is provided with the unitary unit identification code. Accordingly, when the defects are concentrated in the evaluation results of a specific unitary unit identification code, it is found that a cause of the workpiece 101 resulting in the defective product is in the unitary unit 30 provided with the unitary unit identification code.

Next, one example in which the control system 10 (control device 12) of the present invention is applied to a pre-cutting device as the processing unit 20 to be used in the production process of the absorbent article will be described with reference to FIG. 7. The pre-cutting device is provided for cutting an elastic body arranged on a sheet.

As shown in FIG. 7, elastic body groups 213, 214 and 215 formed of a plurality of elastic bodies interposed between two sheets 211 and 212 are cut (pre-cut) and inspected after cutting in several cases. A pre-cutting device 221 has a rotationally-movable pre-cutting roll 222, and the pre-cutting roll 222 has pre-cutting blades 223, 224 and 225. Moreover, a rotatable anvil roll 226 is arranged with facing the pre-cutting roll 222. Such a pre-cutting device 221 includes a multiple-piece unit, in which a large number of times of defects possibly occur only in a specific site. For example, when focusing on pre-cutting of the elastic body group 214, the image pickup section 41 of the inspection section 40 for inspecting a pre-cut part is arranged. Although not shown, a lighting section is arranged in a position facing the image pickup section 41 so as to interpose two sheets 211 and 212 therebetween. Moreover, although not shown, as described above in FIG. 1 described above, the quality of the pre-cut part can be evaluated by the inspection section 40 on the basis of the image picked up by the image pickup section 41.

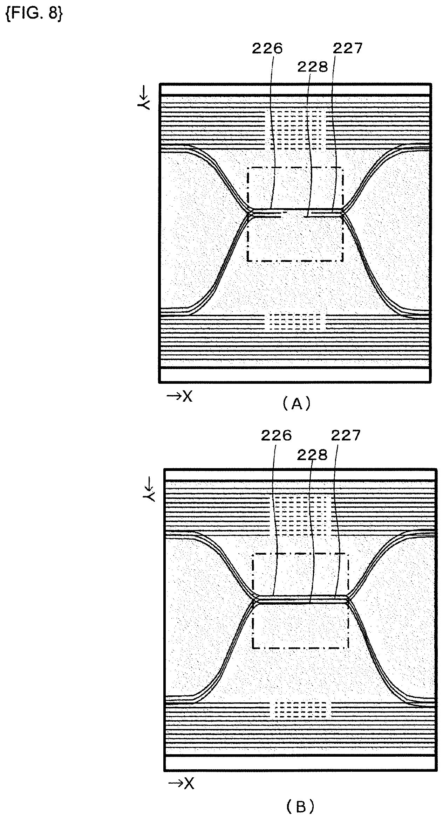

Specifically, consider a case where the elastic body group 214 between areas in which leg holes of the absorbent article are formed is pre-cut by using a pre-cutting blade 224. For example, as shown in FIGs. 8(A) and 8(B), cutting of three elastic bodies 226, 227 and 228 of the elastic body group 214 is insufficient in several cases. In the example shown in FIG. 8(A), the elastic body 226 is not cut, and the elastic bodies 227 and 228 are cut. In such a case, the pre-cutting blade 224 (see FIG. 7) has a nick in several cases. Alternatively, the pre-cutting roll 222 and the anvil roll 226 are possibly not assembled in parallel to each other. Moreover, in the example shown in FIG. 8(B), none of the elastic bodies 226, 227 and 228 is cut. In such a case, examples include insufficient pressing force of the pre-cutting roll 222 (see FIG. 7). Alternatively, an interval (clearance) between the pre-cutting roll 222 and the anvil roll 226 is possibly excessively wide.

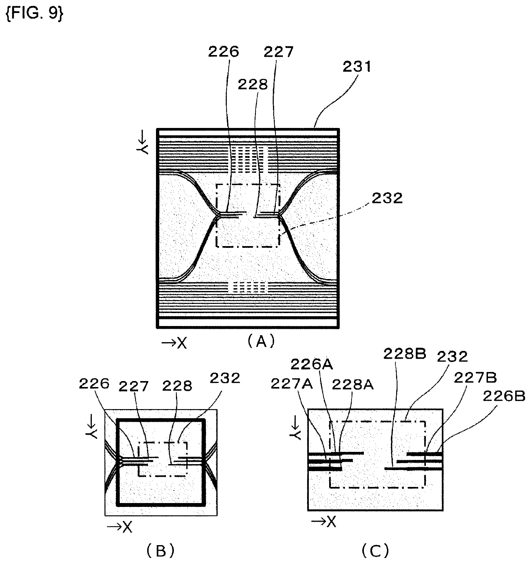

On the other hand, in normal pre-cutting, as shown in FIG. 9(A), three elastic bodies 226, 227 and 228 of the elastic body group 214 are sufficiently cut.

For judging whether or not cutting is performed as described above, as shown in FIG. 9(B), an inspection region 232 is set inside an image pickup area 231 (see FIG. 9(A)). The image processing section 43 binarizes an image inside the inspection region 232 by using a preset binarized threshold to convert the elastic bodies 226, 227 and 228 into black. Then, as shown in FIG. 9(C), lengths (L_{226A}, L_{226B}, L_{227A}, L_{227B}, L_{228A} and L_{228B}) in an x direction inside the inspection region 232 are measured on the images of the elastic bodies 226, 227 and 228 detected by binarization. When the length of each image of the elastic body in the x direction is longer than a reference length, the elastic body is not cut, and therefore such an elastic body is judged defective by the evaluation section 50. When the length of each image of the elastic body in the x direction is less than the reference length, such an elastic body is judged normally cut.

Then, according to the data processing section 60, the number of times when defective cutting occurs is stored in the storage section 70 collectively for each unitary unit identification code. Moreover, according to the reporting section 80, the operator is informed of the necessity to adjust the unitary unit having a large number of times of defects. The operator can fix the fault by changing pressure of the pre-cutting device, or adjusting the clearance, for example.

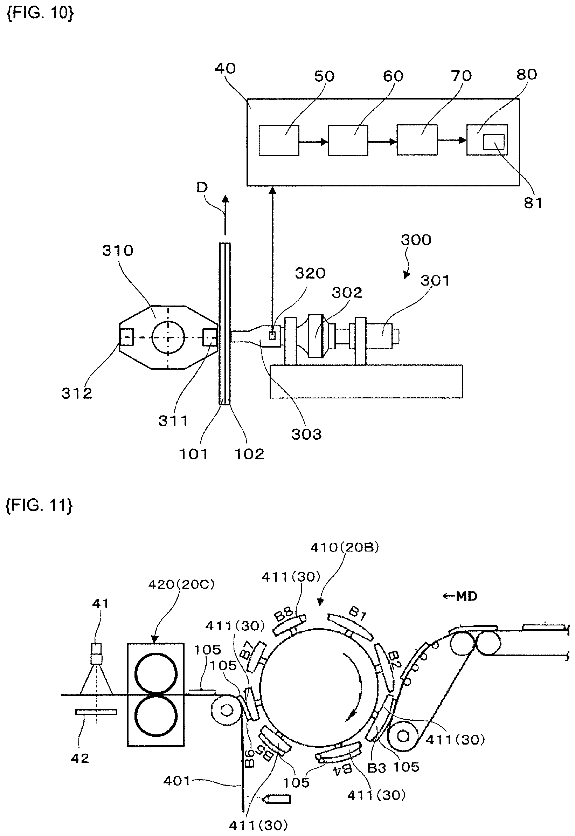

A strain gauge can be used for a sensor to be used in the control system 10 (control device 12) of the production line according to the present invention. For example, as shown in FIG. 10, the strain gauge can be applied to ultrasonic welding equipment 300 as a processing unit 20 having two pattern anvils 311 and 312 on a pattern roll 310. The ultrasonic welding equipment 300 has a converter 301, a booster 302 and a horn 303. The equipment 300 also has the pattern roll 310 in which the pattern anvils 311 and 312 are arranged in the order by rotation of the roll in a position facing the horn 303. The pattern anvil 311 is provided with a unitary unit identification code 1, and the pattern anvil 312 is provided with a unitary unit identification code 2. The converter 301 is provided for converting an ultrasonic wave emitted by an ultrasonic wave oscillator into high frequency electrical energy to convert the energy into mechanical vibration energy by a piezo-electric element. The booster 302 amplifies the mechanical vibration energy. This amplified mechanical vibration energy is transmitted through the horn 303, which is a resonator, to the workpieces 101 and 102 to be welded to weld the workpieces 101 and 102 with each other. Welding is performed by rubbing the workpieces 101 and 102 against each other by the transmitted vibration energy to generate strong frictional heat on a boundary surface to melt the workpieces 101 and 102 with each other. Then, the workpieces 101 and 102 are delivered in an arrow D direction, and moved to the next welding position, and welded again as described above.

During welding, a stress signal of a strain gauge 320 attached to the horn 303 of the ultrasonic welding equipment 300 is input into the evaluation section 50 of the inspection section 40. In this case, the strain gauge 320 plays a role of substitutes for the image pickup section and the lighting section of the inspection section 40. Then, the evaluation section 50 evaluates whether or not a pressure difference is observed when the workpiece is processed by using the pattern anvils 311 and 312 for each unitary unit identification code. Further, the data processing section 60 associates the evaluation results obtained by the evaluation section 50 with the unitary unit identification codes of the unitary unit 30 (pattern anvils 311and 312) in which the evaluated workpieces 101 and 102 are processed. Then, according to the storage section 70, the evaluation results are stored for each unitary unit identification code. Thus, peaks of pressure generated in the pattern anvils 311 and 312 for each unitary unit identification code measured by the strain gauge 320 can be compared. Then, when a difference is observed in the peaks of pressure between the pattern anvils 311 and 312 provided with the unitary unit identification codes 1 and 2, clearance adjustment of the pattern anvils is required. Therefore, according to the display section 81 of the reporting section 80, the unitary unit identification code of the anvil 311 or 312 having the fault, and an effect of presence of pressure abnormality during welding are displayed to urge such clearance adjustment to the operator. Specific examples of an adjustment method include changing of a thickness of a shim (not shown) to be inserted between the pattern roll 310, and the pattern anvils 311 and 312.

The control system 10 (control device 12) of a production line according to the present invention can also be applied to quality evaluation of processing in each processing unit in inspecting a semi-finished product processed by using a plurality of processing units.

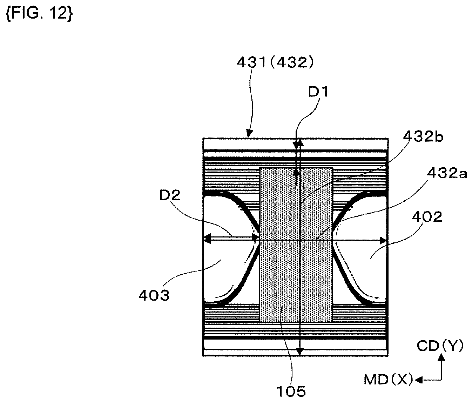

A continuous body 106 of the absorbent bodies 105 is prepared by using the production apparatus 110 (processing unit 20 (also referred to as a processing unit 20A)) shown in FIG. 1 described above. Then, although not shown, the continuous body 106 is cut, and formed into individual absorbent bodies 105. Moreover, as shown in FIG. 11, the absorbent body 105, which is a half-finished product, is formed into a state of being sucked onto an absorbent body attaching pad 411 by a rotary attaching machine 410 (processing unit 20B), and rotated by 90° in a direction thereof, and attached onto a continuous body 401 of exterior packages, which is a half-finished product. Then, the absorbent bodies 105 attached onto the continuous body 401 of the exterior packages are passed through a leg hole cutter 420 (processing unit 20C) to form leg holes 402 (see FIG. 12) in the continuous body 401 of the exterior packages by a cutter (not shown) provided on the leg hole cutter 420. Accordingly, the production apparatus 110, the rotary attaching machine 410 and the leg hole cutter 420 are arranged in this order, and the image pickup section 41 and the lighting section 42 of the inspection section 40 are arranged on a side downstream of the leg hole cutter 420. This inspection section 40 is similar to the section described in FIG. 2 described above. A term "side downstream thereof means a side of downstream of the continuous body 401 in a flow direction.

The production apparatus 110 (processing unit 20A) shown in FIG. 1 has the unitary unit 30 (fiber-stacking recess 141). To the fiber-stacking recess 141, although not shown, unitary unit identification codes A1 to A8 (corresponding to the unitary unit identification codes 1 to 8 in FIG. 1) are assigned, for example. Moreover, the rotary attaching machine 410 (processing unit 20B) shown in FIG. 11 has the plurality of unitary units 30 (absorbent body attaching pads 411), and to the absorbent body attaching pads 411, unitary unit identification codes B1 to B8 are assigned, for example.

Thus, processing is performed by using a plurality of processing units 20A, 20B and 20 C, and then the image of the workpiece 101 is picked up by using the image pickup section 41, whereby the image of the workpiece 101 can be obtained. Therefore, the quality of the workpiece 101 can be evaluated on the basis of the image obtained by the image pickup section 41.

Evaluation is performed as described below, for example.

As shown in FIG. 12, image pickup conditions are set in such a manner that the continuous body 401 of exterior packages in a crosswise direction (CD) enters within a picked-up image 431, and one piece of the absorbent article (semi-finished product) enters therein with the absorbent body 105 as a center in a machine direction (MD) of the continuous body 401 of the exterior packages (see FIG. 11). Examples of the image pickup conditions include an angle of view and image pickup timing. Accordingly, under a normal state, an image of a left half of a leg hole 402 and an image of a right half of a leg hole 403, in which the leg holes 402 and 403 are formed so as to interpose the attached absorbent body 105, are picked up within the picked-up image 431.

First, in order to inspect whether or not the absorbent body 105 is attached on a normal position, it is necessary to measure attachment positions in the MD direction and the CD direction, respectively.

The image processing section 43 sets, for the picked-up image 431, an inspection region 432a for measuring the attachment position in the MD direction (X direction), which is shown by an arrow, and an inspection region 432b for measuring the attachment position in the CD direction (Y direction), which is shown by an arrow. Then, edge extraction is performed on the inspection region 432a in the Y direction, and on the inspection region 432b in the X direction. According to edge extraction processing, measurement is made on an attachment position D1 of the absorbent body in the CD direction, which is detected in the inspection region 432a, and an attachment position D2 of the absorbent body in the MD direction, which is detected in the inspection region 432b. In addition, on a drawing, the inspection region 432a and the attachment position 2D are separately described for better visual understanding, but directions are actually the same.

A term "edge extraction" described above means processing of differentiating the gradation values in the X or Y direction to extract a position in which a change in the gradation value is large, in which various known edge extraction processing can be applied thereto, in addition thereto.

The unitary unit identification code of the attaching pad 411 attached with the absorbent body 105 corresponds to a distance from an image pickup position to a position in which the absorbent body 105 is attached thereon. That is, the unitary unit identification code can be specified according to the number of sheets of the semi-finished products in a length in the MD direction and a rotation angle of the attaching pad 411 when the absorbent body 105 is attached thereon. Accordingly, the data processing section 60 (see FIG. 2) can associate the picked-up image picked up by the image processing section 43 with the unitary unit identification codes B1 to B8 (see FIG. 11).

Then, the data processing section 60 summarizes measurement data for each unitary unit identification code. Moreover, a standard deviation of the attachment positions of the absorbent bodies 105, which is necessary for evaluating whether or not attachment accuracy of the absorbent body 105 in the rotary attaching machine 410 (processing unit 20B) is different for each unitary unit identification code, can also be determined, respectively.

Moreover, the evaluation section 50 can receive the standard deviation of the attachment positions by each absorbent body attaching pad 411 from the data processing section 60. Then, the evaluation section 50 evaluates whether or not the standard deviation of the attachment positions of the absorbent bodies 105 for each unitary unit identification code is larger than a preset [deviation threshold]. When the standard deviation of the attachment positions of the absorbent bodies 105 is more than the deviation threshold, any abnormality is judged to occur. Thus, in the step of obtaining the evaluation results by the evaluation section 50, the standard deviation is determined from the data for each unitary unit identification code, and any abnormality is evaluated from the above-described standard deviation.

Then, the data processing section 60 allows the evaluation results to correspond to an attachment abnormality flag and the unitary unit identification code in which any attachment abnormality occurs, and to store the correspondence in the storage section 70. At the same time, the correspondence is transmitted to the reporting section 80 and the control section 90 as the detected information.

The reporting section 80 allows the message based on the attachment position abnormality flag to display on the display section 81 to urge caution to the operator.

The control section 90 can also perform control for increasing suction pressure of a suction device (not shown) installed to the absorbent body attaching pad 411, for example, in order to reduce the variation of the attachment positions of the absorbent bodies 105.

Further, when the suction pressure is more than an allowance, the control section 90 allows the production line to stop. At this time, the control section 90 transmits a signal indicating that the suction pressure is more than the allowance, and the unitary unit identification code of the absorbent body attaching pad 411 having any abnormality to the reporting section 80. The reporting section 80 allows the message suited to the content to display on the display section 81 to urge inspection to the operator. According to the above-described operation, attachment accuracy of the absorbent body 105 can be improved.

Moreover, with regard to the above-described image picked up, an inspection region (not shown) is provided in a part of the absorbent body 105, and the image is binarized, whereby the minute chipping can be detected as described above with reference to FIGs. 1 to 4. Further, the gradation value of the absorbent body 105 is determined as described above with reference to FIGs. 5 to 6, whereby any basis weight abnormality of the absorbent body 105 can be detected. Thus, the minute chipping and any basis weight abnormality should be detected as mentioned above.

Moreover, according to the storage section 70, the evaluation results are stored for each of the unitary unit identification codes A1 to A8 and B1 to B8. At the same time, in the reporting section 80, the unitary unit identification codes and the evaluation results are reported. Then, the unitary unit identification codes associated with the evaluation results, and the evaluation results are displayed on the display section 81.

Thus, the evaluation results are obtained for each of the unitary unit identification codes A1 to A8 and B1 to B8, and therefore in such a case where the evaluation results corresponding to a certain unitary unit identification code are more than the threshold, such a case is evaluated abnormal in the unitary unit 30 having the unitary unit identification code.

Thus, even if the image is picked up only once on one workpiece 101 by one image pickup section 41, the unitary units 30 of the plurality of processing units 20 can be simultaneously inspected.

With respect to the above-mentioned embodiments, the present invention further discloses the following aspects.
<1> A control system of a production line, containing a processing unit from which a workpiece processed is obtained,
   wherein the control system has:
   the processing unit; and
   a control unit for controlling the processing unit,
   wherein the processing unit has a plurality of unitary units,
   wherein each of the plurality of unitary units has a unitary unit identification code,
   wherein the control system of a production line has an inspection section for inspecting the workpiece processed,
   wherein the inspection section can communicate inside the control system of a production line, and
   wherein the inspection section has an evaluation section in which state of the processed workpiece is evaluated to obtain evaluation results; a data processing section in which the evaluation results are associated with the unitary unit identification codes of the processing unit in which the evaluated workpiece is processed; a storage section in which the evaluation results are stored for each of the unitary unit identification codes; and a reporting section by which the unitary unit identification codes and the evaluation results are reported.
<2> The control system of a production line as described in the above item <1>,
   wherein the inspection section has:
   an image pickup section for picking up an image of the workpiece processed by the processing unit, and
   an image processing section for processing the image picked up by the image pickup section; and
   wherein the evaluation section evaluates state of the workpiece produced by the processing unit by comparing processed data obtained by the image processing section and pre-registered normal product processed data.
<3> The control system of a production line as described in the above item <2>, wherein the image pickup section picks up a transmitted image of an absorbent body.
<4> The control system of a production line as described in the above item <2> or <3>, wherein the image processing section binarizes the picked up image obtained by the image pickup section using a preset binarized threshold.
<5> The control system of a production line as described in any one of the above items <2> to <4>, wherein the image processing section preliminary prepares a calibration curve representing a relationship between a basis weight and a gradation value of an absorbent body from a picked-up image of the absorbent body to determine an allowable range of the gradation value from an allowable range of the basis weight of the absorbent body.
<6> The control system of a production line as described in any one of the above items <1> to <5>, wherein the reporting section has a display section for displaying the unitary unit identification codes associated with the evaluation results, and the evaluation results.
<7> The control system of a production line as described in any one of the above items <1> to <6>, wherein the reporting section is a sound output section for reporting the unitary unit identification codes associated with the evaluation results, and the evaluation results by sound.
<8> The control system of a production line as described in any one of the above items <1> to <7>, wherein the data processing section determines a standard deviation from data for each of the unitary unit identification codes to transmit the standard deviation to the evaluation section, and the evaluation section evaluates abnormality from the standard deviation.
<9> The control system of a production line as described in any one of the above items <1> to <8>, containing a control section for allowing production conditions of the processing unit to change or allowing the production line to stop on the basis of the evaluation results associated with the unitary unit identification codes,
   wherein the control section can communicate inside the control system of a production line.
<10> The control system of a production line as described in the above item <9>, wherein the evaluation section counts the number of pixels converted into black, in which a pixel lower than a binarized threshold T_{B} is converted into black and a pixel more than the threshold T_{B} is converted into white; judges whether or not the number of black pixels counted is within a range of a preset lower limit threshold T_{PL} of the preset number of pixels and a preset upper limit threshold T_{PH} thereof; evaluates whether or not a workpiece an image of which is picked up is normal; and outputs quality evaluation data to the control section.
<11> The control system of a production line as described in any one of the above items <1> to <10>,
   wherein the production line contains an ejection unit, and
   wherein the control system of a production line allows the ejection unit to operate on the basis of the evaluation results.
<12> The control system of a production line as described in any one of the above items <1> to <11>,
   wherein the production line contains a plurality of the processing units having different functions,
   wherein the processing unit has a plurality of the unitary units, wherein the plurality of the unitary units have unitary unit identification codes, respectively, and
   wherein the inspection section in which the workpiece processed by the plurality of the processing units is inspected is arranged.
<13> The control system of a production line as described in any one of the above items <1> to <12>, wherein the processing unit is an apparatus for producing an absorbent body.
<14> The control system of a production line as described in the above item <13>, wherein the apparatus for producing an absorbent body is provided for depositing an absorbent material containing a fiber material into a fiber-staking recess of a fiber stacker.
<15> The control system of a production line as described in the above item <14>, wherein the workpiece is an absorbent body deposited in the fiber-stacking recess.
<16> The control system of a production line as described in the above item <14> or <15>, wherein the unitary unit is a plurality of fiber-stacking recesses in the fiber stacker of the apparatus for producing an absorbent body.
<17> The control system of a production line as described in any one of the above items <1> to <16>,
   wherein the data processing section calculates the total average value ((N-m) × n pixels) of the number of black pixels and the average value of the number of black pixels for each unitary unit identification code, to determine a differential between the total average value of the number of black pixels and the average value of the number of black pixels in each unitary unit identification code ([total average value] - [average value in each unitary unit]), and
   wherein the evaluation section judges whether or not the differential value for each unitary unit identification code is within the range of a preset lower limit threshold TS_{PL} and a preset upper limit threshold TS_{PH}.
<18> The control system of a production line as described in any one of the above items <1> to <17>, wherein the evaluation section evaluates such a case normal when the value is within the range between a preset lower limit threshold T_{GL} of the average gradation value and a preset upper limit threshold T_{GH} thereof, and such a case abnormal in the basis weight of an absorbent body when the value is out of the range.
<19> The control system of a production line as described in any one of the above items <1> to <18>,
   wherein the data processing section is provided for calculating a differential between a total average value of gradation values of absorbent bodies and an average value of the gradation values in the unitary unit identification codes ([total average value] - [average gradation value in each unitary unit]), and
   wherein the evaluation section is provided for judging whether or not the calculated differential value is out of a preset lower limit threshold TS_{GL} of a differential and a preset upper limit threshold TS_{GH} thereof.
<20> The control system of a production line as described in any one of the above items <1> to <19>, wherein the control section allows a production condition of the processing unit requiring the change in the production condition to change on the basis of the detected information transmitted from the inspection section.
<21> The control system of a production line as described in any one of the above items <1> to <20>, wherein the processing unit has a rotary attaching machine for cutting a continuous body of absorbent bodies, rotating an individually formed absorbent body by 90° in a direction to attach the absorbent body onto a continuous body of exterior packages, which is a semi-finished product.
<22> A control device of a production line, containing a processing unit from which a workpiece processed is obtained,
   wherein the control device has:
   the processing unit; and
   a control unit for controlling the processing unit,
   wherein the processing unit has a plurality of unitary units,
   wherein each of the plurality of unitary units has a unitary unit identification code,
   wherein the control unit has an inspection section for inspecting the workpiece obtained from the processing unit,
   wherein the inspection section can communicate inside the control system of a production line, and
   wherein the inspection section has an evaluation section in which state of the processed workpiece is evaluated to obtain evaluation results; a data processing section in which the evaluation results are associated with the unitary unit identification codes of the processing unit in which the evaluated workpiece is processed; a storage section in which the evaluation results are stored for each of the unitary unit identification codes; and a reporting section in which the unitary unit identification codes and the evaluation results are reported.
<23> The apparatus for controlling a production line as described in the above item <22>,
   wherein the inspection section has:
   an image pickup section for picking up an image of the workpiece processed by the processing unit, and
   an image processing section for processing the image picked up by the image pickup section; and
   wherein the evaluation section evaluates state of the workpiece produced by the processing unit by comparing processed data obtained by the image processing section and pre-registered normal product processed data.
<24> The apparatus for controlling a production line as described in the above item <23>, wherein the image pickup section picks up a transmitted image of an absorbent body.
<25> The apparatus for controlling a production line as described in the above item <23> or <24>, wherein the image processing section binarizes the picked up image obtained by the image pickup section using a preset binarized threshold.
<26> The apparatus for controlling a production line as described in any one of the above items <23> to <25>, wherein the image processing section preliminary prepares a calibration curve representing a relationship between a basis weight and a gradation value of an absorbent body from a picked-up image of the absorbent body to determine an allowable range of the gradation value from an allowable range of the basis weight of the absorbent body.
<27> The apparatus for controlling a production line as described in any one of the above items <22> to <26>, wherein the reporting section has a display section for displaying the unitary unit identification codes associated with the evaluation results, and the evaluation results.
<28> The apparatus for controlling a production line as described in any one of the above items <22> to <27>, wherein the reporting section is a sound output section for reporting the unitary unit identification codes associated with the evaluation results, and the evaluation results by sound.
<29> The apparatus for controlling a production line as described in any one of the above items <22> to <28>, wherein the data processing section determines a standard deviation from data for each of the unitary unit identification codes to transmit the standard deviation to the evaluation section, and the evaluation section evaluates abnormality from the standard deviation.
<30> The apparatus for controlling a production line as described in any one of the above items <22> to <29>, containing a control section for allowing production conditions of the processing unit to change or allowing the production line to stop on the basis of the evaluation results associated with the unitary unit identification codes,
   wherein the control section can communicate inside the control system of a production line.
<31> The apparatus for controlling a production line as described in the above item <30>, wherein the evaluation section counts the number of pixels converted into black, in which a pixel lower than a binarized threshold T_{B} is converted into black and a pixel more than the threshold T_{B} is converted into white; judges whether or not the number of black pixels counted is within a range of a preset lower limit threshold T_{PL} of the preset number of pixels and a preset upper limit threshold T_{PH} thereof; evaluates whether or not a workpiece an image of which is picked up is normal; and outputs quality evaluation data to the control section.
<32> The apparatus for controlling a production line as described in any one of the above items <22> to <31>,
   wherein the production line contains an ejection unit, and
   wherein the apparatus for controlling a production line allows the ejection unit to operate on the basis of the evaluation results.
<33> The apparatus for controlling a production line as described in any one of the above items <22> to <32>,
   wherein the production line contains a plurality of the processing units having different functions,
   wherein the processing unit has a plurality of the unitary units,
   wherein the plurality of the unitary units have unitary unit identification codes, respectively, and
   wherein the inspection section in which the workpiece processed by the plurality of the processing units is inspected is arranged.
<34> The apparatus for controlling a production line as described in any one of the above items <22> to <33>, wherein the processing unit is an apparatus for producing an absorbent body.
<35> The apparatus for controlling a production line as described in the above item <34>, wherein the apparatus for producing an absorbent body is provided for depositing an absorbent material containing a fiber material into a fiber-staking recess of a fiber stacker.
<36> The apparatus for controlling a production line as described in the above item <35>, wherein the workpiece is an absorbent body deposited in the fiber-stacking recess.
<37> The apparatus for controlling a production line as described in the above item <35> or <36>, wherein the unitary unit is a plurality of fiber-stacking recesses in the fiber stacker of the apparatus for producing an absorbent body.
<38> The apparatus for controlling a production line as described in any one of the above items <22> to <37>,
   wherein the data processing section calculates the total average value ((N-m) × n pixels) of the number of black pixels and the average value of the number of black pixels for each unitary unit identification code, to determine a differential between the total average value of the number of black pixels and the average value of the number of black pixels in each unitary unit identification code ([total average value] - [average value in each unitary unit]), and
   wherein the evaluation section judges whether or not the differential value for each unitary unit identification code is within the range of a preset lower limit threshold TS_{PL} and a preset upper limit threshold TS_{PH}.
<39> The apparatus for controlling a production line as described in any one of the above items <22> to <38>, wherein the evaluation section evaluates such a case normal when the value is within the range between a preset lower limit threshold T_{GL} of the average gradation value and a preset upper limit threshold T_{GH} thereof, and such a case abnormal in the basis weight of an absorbent body when the value is out of the range.
<40> The apparatus for controlling a production line as described in any one of the above items <22> to <39>,
   wherein the data processing section is provided for calculating a differential between a total average value of gradation values of absorbent bodies and an average value of the gradation values in the unitary unit identification codes ([total average value] - [average gradation value in each unitary unit]), and
   wherein the evaluation section is provided for judging whether or not the calculated differential value is out of a preset lower limit threshold TS_{GL} of a differential and a preset upper limit threshold TS_{GH} thereof.
<41> The apparatus for controlling a production line as described in any one of the above items <22> to <40>, wherein the control section allows a production condition of the processing unit requiring the change in the production condition to change on the basis of the detected information transmitted from the inspection section.
<42> The apparatus for controlling a production line as described in any one of the above items <22> to <41>, wherein the processing unit has a rotary attaching machine for cutting a continuous body of absorbent bodies, rotating an individually formed absorbent body by 90° in a direction to attach the absorbent body onto a continuous body of exterior packages, which is a semi-finished product.
<43> A method of controlling a production line having a plurality of processing units for producing a workpiece processed, containing the steps of:
   assigning a unitary unit identification code to each of a plurality of unitary units of the processing unit;
   inspecting the produced workpiece;
   evaluating state of the inspected workpiece to obtain evaluation results;
   associating the evaluation results with the unitary unit identification codes of the processing unit in which the evaluated workpiece is processed;
   storing the evaluation results for each of the unitary unit identification codes; and
   reporting the unitary unit identification codes and the evaluation results.
<44> The method of controlling a production line as described in the above item <43>, wherein, in the step for obtaining the evaluation results,
   a picked-up image obtained by picking up the image of the workpiece produced by the processing unit, by the image pickup section, is subjected to image processing by the image processing section; and
   the processed data obtained by this image processing is compared with the pre-registered normal product processed data to evaluate the state of the workpiece produced by the processing unit.
<45> The method of controlling a production line as described in the above item <44>,
   wherein the workpiece is an absorbent body, and
   wherein the image pickup section picks up a transmitted image of the absorbent body.
<46> The method of controlling a production line as described in the above item <45>, wherein the image processing section preliminary prepares a calibration curve representing a relationship between a basis weight and a gradation value of an absorbent body from a picked-up image of the absorbent body to determine an allowable range of the gradation value from an allowable range of the basis weight of the absorbent body.
<47> The method of controlling a production line as described in any one of the above items <44> to <46>, wherein the image processing section binarizes the picked up image obtained by the image pickup section using a preset binarized threshold.
<48> The method of controlling a production line as described in any one of the above items <43> to <47>, wherein, in the reporting step, the unitary unit identification codes associated with the evaluation results, and the evaluation results are reported.
<49> The method of controlling a production line as described in any one of the above items <43> to <48>, wherein, in the reporting step, the unitary unit identification codes associated with the evaluation results, and the evaluation results are reported by sound.
<50> The method of controlling a production line as described in any one of the above items <43> to <49>, wherein, in a step of obtaining the evaluation results, a standard deviation is determined from data for each of the unitary unit identification codes to evaluate abnormality from the standard deviation.
<51> The method of controlling a production line as described in any one of the above items <43> to <50>, wherein production conditions of the processing unit are changed or the production line is stopped on the basis of the evaluation results associated with the unitary unit identification codes.
<52> The method of controlling a production line as described in any one of the above items <43> to <51>, wherein a defective product is ejected from the production line on the basis of the evaluation results.
<53> The method of controlling a production line as described in any one of the above items <43> to <52>, wherein the plurality of processing units is controlled.
<54> The method of controlling a production line as described in any one of the above items <43> to <53>, wherein the processing unit is an apparatus for producing an absorbent body.
<55> The method of controlling a production line as described in the above item <54>, wherein the apparatus for producing an absorbent body is provided for depositing an absorbent material containing a fiber material into a fiber-staking recess of a fiber stacker.
<56> The method of controlling a production line as described in the above item <55>, wherein the workpiece is an absorbent body deposited in the fiber-stacking recess.
<57> The method of controlling a production line as described in the above item <55> or <56>, wherein the unitary unit is a plurality of fiber-stacking recesses in the fiber stacker of the apparatus for producing an absorbent body.

Having described our invention as related to this embodiments, it is our intention that the invention not be limited by any of the details of the description, unless otherwise specified, but rather be construed broadly within its spirit and scope as set out in the accompanying claims.

This application claims priority on Patent Application No. 2016-235063 filed in Japan on December 2, 2016, and Patent Application No. 2017-226718 filed in Japan on November 27, 2017, each of which is entirely herein incorporated by reference.

### DESCRIPTION OF SYMBOLS

10 Control system of production line
11 Control unit
12 Control device
20, 20A, 20B, 20C Processing unit
30 Unitary unit
40 Inspection section
41 Image pickup section
42 Lighting section
43 Image processing section
50 Evaluation section
60 Data processing section
70 Storage section
80 Reporting section
81 Display section
90 Control section
101, 102 Workpiece
105 Absorbent body
105h Minute chipping
106 Continuous body
109 Backing
110 Production apparatus
120 Defibrator
121 Casing
122 Rotating blade
123, 124 Opening portion
130 Duct
130a One end portion
130b Other end portion
140 Fiber stacker
141 Fiber-stacking recess
142 Rotating drum
150 Pulp feeder
151 Pulp sheet
152 Pulp fiber
153 Absorbent polymer
154 Absorbent material
170 Conveying device
211, 212 Sheet
213, 214, 215 Elastic body group
221 Pre-cutting device
222 Pre-cutting roll
223, 224, 225 Pre-cutting blade
226 Anvil roll
226, 227, 228 Elastic body
226A, 226B, 228A, 228B Image of elastic body
231 Picked-up image
232 Inspection region
300 Ultrasonic welding equipment
301 Converter
302 Booster
303 Horn
310 Pattern roll
311, 312 Pattern anvil
320 Strain gauge
401 Continuous body of exterior packages
410 Rotary attaching machine
411 Absorbent body attaching pad
420 Leg hole cutter
431 Picked-up image
432, 432a, 432b Inspection region
B, C, D Space

## Claims

1. A control system of a production line, comprising a processing unit from which a workpiece processed is obtained,
wherein the control system comprises:
the processing unit; and
a control unit for controlling the processing unit,
wherein the processing unit comprises a plurality of unitary units,
wherein each of the plurality of unitary units has a unitary unit identification code,
wherein the control system of a production line comprises an inspection section for inspecting the workpiece processed,
wherein the inspection section can communicate inside the control system of a production line, and
wherein the inspection section comprises an evaluation section in which state of the workpiece processed is evaluated to obtain evaluation results; a data processing section in which the evaluation results are associated with the unitary unit identification codes of the processing unit in which the evaluated workpiece is processed; a storage section in which the evaluation results are stored for each of the unitary unit identification codes; and a reporting section by which the unitary unit identification codes and the evaluation results are reported.

2. The control system of a production line according to Claim 1,
wherein the inspection section comprises:
an image pickup section for picking up an image of the workpiece processed by the processing unit, and
an image processing section for processing the image picked up by the image pickup section; and
wherein the evaluation section evaluates state of the workpiece produced by the processing unit by comparing processed data obtained by the image processing section and pre-registered normal product processed data.

3. The control system of a production line according to Claim 2, wherein the image pickup section picks up a transmitted image of an absorbent body.

4. The control system of a production line according to Claim 2 or 3,
wherein the image processing section binarizes the picked up image obtained by the image pickup section using a preset binarized threshold.

5. The control system of a production line according to any one of Claims 2 to 4, wherein the image processing section preliminary prepares a calibration curve representing a relationship between a basis weight and a gradation value of an absorbent body from a picked-up image of the absorbent body to determine an allowable range of the gradation value from an allowable range of the basis weight of the absorbent body.

6. The control system of a production line according to any one of Claims 1 to 5, wherein the reporting section comprises a display section for displaying the unitary unit identification codes associated with the evaluation results, and the evaluation results.

7. The control system of a production line according to any one of Claims 1 to 6, wherein the reporting section is a sound output section for reporting the unitary unit identification codes associated with the evaluation results, and the evaluation results by sound.

8. The control system of a production line according to any one of Claims 1 to 7, wherein the data processing section determines a standard deviation from data for each of the unitary unit identification codes to transmit the standard deviation to the evaluation section, and the evaluation section evaluates abnormality from the standard deviation.

9. The control system of a production line according to any one of Claims 1 to 8, comprising a control section for allowing production conditions of the processing unit to change or allowing the production line to stop on the basis of the evaluation results associated with the unitary unit identification codes, wherein the control section can communicate inside the control system of a production line.

10. The control system of a production line according to Claim 9, wherein the evaluation section counts the number of pixels converted into black, in which a pixel lower than a binarized threshold T_{B} is converted into black and a pixel more than the threshold T_{B} is converted into white; judges whether or not the number of black pixels counted is within a range of a preset lower limit threshold T_{PL} of the preset number of pixels and a preset upper limit threshold T_{PH} thereof; evaluates whether or not a workpiece an image of which is picked up is normal; and outputs quality evaluation data to the control section.

11. The control system of a production line according to any one of Claims 1 to 10,
wherein the production line comprises an ejection unit, and
wherein the control system of a production line allows the ejection unit to operate on the basis of the evaluation results.

12. The control system of a production line according to any one of Claims 1 to 11,
wherein the production line comprises a plurality of the processing units having different functions,
wherein the processing unit has a plurality of the unitary units,
wherein the plurality of the unitary units have unitary unit identification codes, respectively, and
wherein the inspection section in which the workpiece processed by the plurality of the processing units is inspected is arranged.

13. The control system of a production line according to any one of Claims 1 to 12, wherein the processing unit is an apparatus for producing an absorbent body.

14. The control system of a production line according to Claim 13, wherein the apparatus for producing an absorbent body is provided for depositing an absorbent material containing a fiber material into a fiber-staking recess of a fiber stacker.

15. The control system of a production line according to Claim 14, wherein the workpiece is an absorbent body deposited in the fiber-stacking recess.

16. The control system of a production line according to Claim 14 or 15,
wherein the unitary unit is a plurality of fiber-stacking recesses in the fiber stacker of the apparatus for producing an absorbent body.

17. The control system of a production line according to any one of Claims 1 to 16,
wherein the data processing section calculates the total average value ((N-m) × n pixels) of the number of black pixels and the average value of the number of black pixels for each unitary unit identification code, to determine a differential between the total average value of the number of black pixels and the average value of the number of black pixels in each unitary unit identification code ([total average value] - [average value in each unitary unit]), and
wherein the evaluation section judges whether or not the differential value for each unitary unit identification code is within the range of a preset lower limit threshold TS_{PL} and a preset upper limit threshold TS_{PH}.

18. The control system of a production line according to any one of Claims 1 to 17, wherein the evaluation section evaluates such a case normal when the value is within the range between a preset lower limit threshold T_{GL} of the average gradation value and a preset upper limit threshold T_{GH} thereof, and such a case abnormal in the basis weight of an absorbent body when the value is out of the range.

19. The control system of a production line according to any one of Claims 1 to 18,
wherein the data processing section is provided for calculating a differential between a total average value of gradation values of absorbent bodies and an average value of the gradation values in the unitary unit identification codes ([total average value] - [average gradation value in each unitary unit]), and
wherein the evaluation section is provided for evaluating whether or not the calculated differential value is out of a preset lower limit threshold TS_{GL} of a differential and a preset upper limit threshold TS_{GH} thereof.

20. The control system of a production line according to any one of Claims 1 to 19, wherein the control section allows a production condition of the processing unit requiring the change in the production condition to change on the basis of the detected information transmitted from the inspection section.

21. The control system of a production line according to any one of Claims 1 to 20, wherein the processing unit has a rotary attaching machine for cutting a continuous body of absorbent bodies, rotating an individually formed absorbent body by 90° in a direction to attach the absorbent body onto a continuous body of exterior packages, which is a semi-finished product.

22. A control device of a production line, comprising a processing unit from which a workpiece processed is obtained,
wherein the control device comprises:
the processing unit; and
a control unit for controlling the processing unit,
wherein the processing unit comprises a plurality of unitary units,
wherein each of the plurality of unitary units has a unitary unit identification code,
wherein the control unit comprises an inspection section for inspecting the workpiece obtained from the processing unit,
wherein the inspection section can communicate inside the control system of a production line, and
wherein the inspection section comprises an evaluation section in which state of the processed workpiece is evaluated to obtain evaluation results; a data processing section in which the evaluation results are associated with the unitary unit identification codes of the processing unit in which the evaluated workpiece is processed; a storage section in which the evaluation results are stored for each of the unitary unit identification codes; and a reporting section in which the unitary unit identification codes and the evaluation results are reported.

23. The apparatus for controlling a production line according to Claim 22,
wherein the inspection section comprises:
an image pickup section for picking up an image of the workpiece processed by the processing unit, and
an image processing section for processing the image picked up by the image pickup section; and
wherein the evaluation section evaluates state of the workpiece produced by the processing unit by comparing processed data obtained by the image processing section and pre-registered normal product processed data.

24. The apparatus for controlling a production line according to Claim 23,
wherein the image pickup section picks up a transmitted image of an absorbent body.

25. The apparatus for controlling a production line according to Claim 23 or 24, wherein the image processing section binarizes the picked up image obtained by the image pickup section using a preset binarized threshold.

26. The apparatus for controlling a production line according to any one of Claims 23 to 25, wherein the image processing section preliminary prepares a calibration curve representing a relationship between a basis weight and a gradation value of an absorbent body from a picked-up image of the absorbent body to determine an allowable range of the gradation value from an allowable range of the basis weight of the absorbent body.

27. The apparatus for controlling a production line according to any one of Claims 22 to 26, wherein the reporting section comprises a display section for displaying the unitary unit identification codes associated with the evaluation results, and the evaluation results.

28. The apparatus for controlling a production line according to any one of Claims 22 to 27, wherein the reporting section is a sound output section for reporting the unitary unit identification codes associated with the evaluation results, and the evaluation results by sound.

29. The apparatus for controlling a production line according to any one of Claims 22 to 28, wherein the data processing section determines a standard deviation from data for each of the unitary unit identification codes to transmit the standard deviation to the evaluation section, and the evaluation section evaluates abnormality from the standard deviation.

30. The apparatus for controlling a production line according to any one of Claims 22 to 29, comprising a control section for allowing production conditions of the processing unit to change or allowing the production line to stop on the basis of the evaluation results associated with the unitary unit identification codes, wherein the control section can communicate inside the control system of a production line.

31. The apparatus for controlling a production line according to Claim 30, wherein the evaluation section counts the number of pixels converted into black, in which a pixel lower than a binarized threshold T_{B} is converted into black and a pixel more than the threshold T_{B} is converted into white; judges whether or not the number of black pixels counted is within a range of a preset lower limit threshold T_{PL} of the preset number of pixels and a preset upper limit threshold T_{PH} thereof; evaluates whether or not a workpiece an image of which is picked up is normal; and outputs quality evaluation data to the control section.

32. The apparatus for controlling a production line according to any one of Claims 22 to 31,
wherein the production line comprises an ejection unit, and
wherein the apparatus for controlling a production line allows the ejection unit to operate on the basis of the evaluation results.

33. The apparatus for controlling a production line according to any one of Claims 22 to 32,
wherein the production line comprises a plurality of the processing units having different functions,
wherein the processing unit has a plurality of the unitary units,
wherein the plurality of the unitary units have unitary unit identification codes, respectively, and
wherein the inspection section in which the workpiece processed by the plurality of the processing units is inspected is arranged.

34. The apparatus for controlling a production line according to any one of Claims 22 to 33, wherein the processing unit is an apparatus for producing an absorbent body.

35. The apparatus for controlling a production line according to Claim 34,
wherein the apparatus for producing an absorbent body is provided for depositing an absorbent material containing a fiber material into a fiber-staking recess of a fiber stacker.

36. The apparatus for controlling a production line according to Claim 35,
wherein the workpiece is an absorbent body deposited in the fiber-stacking recess.

37. The apparatus for controlling a production line according to Claim 35 or 36, wherein the unitary unit is a plurality of fiber-stacking recesses in the fiber stacker of the apparatus for producing an absorbent body.

38. The apparatus for controlling a production line according to any one of Claims 22 to 37,
wherein the data processing section calculates the total average value ((N-m) × n pixels) of the number of black pixels and the average value of the number of black pixels for each unitary unit identification code, to determine a differential between the total average value of the number of black pixels and the average value of the number of black pixels in each unitary unit identification code ([total average value] - [average value in each unitary unit]), and
wherein the evaluation section judges whether or not the differential value for each unitary unit identification code is within the range of a preset lower limit threshold TS_{PL} and a preset upper limit threshold TS_{PH}.

39. The apparatus for controlling a production line according to any one of Claims 22 to 38, wherein the evaluation section evaluates such a case normal when the value is within the range between a preset lower limit threshold T_{GL} of the average gradation value and a preset upper limit threshold T_{GH} thereof, and such a case abnormal in the basis weight of an absorbent body when the value is out of the range.

40. The apparatus for controlling a production line according to any one of Claims 22 to 39,
wherein the data processing section is provided for calculating a differential between a total average value of gradation values of absorbent bodies and an average value of the gradation values in the unitary unit identification codes ([total average value] - [average gradation value in each unitary unit]), and
wherein the evaluation section is provided for judging whether or not the calculated differential value is out of a preset lower limit threshold TS_{GL} of a differential and a preset upper limit threshold TS_{GH} thereof.

41. The apparatus for controlling a production line according to any one of Claims 22 to 40, wherein the control section allows a production condition of the processing unit requiring the change in the production condition to change on the basis of the detected information transmitted from the inspection section.

42. The apparatus for controlling a production line according to any one of Claims 22 to 41, wherein the processing unit has a rotary attaching machine for cutting a continuous body of absorbent bodies, rotating an individually formed absorbent body by 90° in a direction to attach the absorbent body onto a continuous body of exterior packages, which is a semi-finished product.

43. A method of controlling a production line having a plurality of processing units for producing a workpiece processed, comprising the steps of:
assigning a unitary unit identification code to each of a plurality of unitary units of the processing unit;
inspecting the produced workpiece;
evaluating state of the inspected workpiece to obtain evaluation results;
associating the evaluation results with the unitary unit identification codes of the processing unit in which the evaluated workpiece is processed;
storing the evaluation results for each of the unitary unit identification codes; and
reporting the unitary unit identification codes and the evaluation results.

44. The method of controlling a production line according to Claim 43,
wherein, in the step for obtaining the evaluation results,
a picked-up image obtained by picking up the image of the workpiece produced by the processing unit, by the image pickup section, is subjected to image processing by the image processing section; and
the processed data obtained by this image processing is compared with the pre-registered normal product processed data to evaluate the state of the workpiece produced by the processing unit.

45. The method of controlling a production line according to Claim 44,
wherein the workpiece is an absorbent body, and
wherein the image pickup section picks up a transmitted image of the absorbent body.

46. The method of controlling a production line according to Claim 45,
wherein the image processing section preliminary prepares a calibration curve representing a relationship between a basis weight and a gradation value of an absorbent body from a picked-up image of the absorbent body to determine an allowable range of the gradation value from an allowable range of the basis weight of the absorbent body.

47. The method of controlling a production line according to any one of Claims 44 to 46, wherein the image processing section binarizes the picked up image obtained by the image pickup section using a preset binarized threshold.

48. The method of controlling a production line according to any one of Claims 43 to 47, wherein, in the reporting step, the unitary unit identification codes associated with the evaluation results, and the evaluation results are reported.

49. The method of controlling a production line according to any one of Claims 43 to 48, wherein, in the reporting step, the unitary unit identification codes associated with the evaluation results, and the evaluation results are reported by sound.

50. The method of controlling a production line according to any one of Claims 43 to 49, wherein, in a step of obtaining the evaluation results, a standard deviation is determined from data for each of the unitary unit identification codes to evaluate abnormality from the standard deviation.

51. The method of controlling a production line according to any one of Claims 43 to 50, wherein production conditions of the processing unit are changed or the production line is stopped on the basis of the evaluation results associated with the unitary unit identification codes.

52. The method of controlling a production line according to any one of Claims 43 to 51, wherein a defective product is ejected from the production line on the basis of the evaluation results.

53. The method of controlling a production line according to any one of Claims 43 to 52, wherein the plurality of processing units is controlled.

54. The method of controlling a production line according to any one of Claims 43 to 53, wherein the processing unit is an apparatus for producing an absorbent body.

55. The method of controlling a production line according to Claim 54,
wherein the apparatus for producing an absorbent body is provided for depositing an absorbent material containing a fiber material into a fiber-staking recess of a fiber stacker.

56. The method of controlling a production line according to Claim 55,
wherein the workpiece is an absorbent body deposited in the fiber-stacking recess.

57. The method of controlling a production line according to Claim 55 or 56,
wherein the unitary unit is a plurality of fiber-stacking recesses in the fiber stacker of the apparatus for producing an absorbent body.
